# EUROPEAN PATENT APPLICATION

(11) **EP 4 736 959 A1**
(43) Date of publication of application: **06.05.2026**
(21) Application number: 24210648.2
(22) Date of filing: 04.11.2024
(51) Int. Cl.: A61Q 5/00, A61K 8/73, A61Q 5/06

(54) **BIODEGRADABLE VEGAN CHITOSAN HYDROCHLORIDE FOR THERMAL HAIR PROTECTION, HAIR DENSITY, AND ANTI-STATIC EFFECT**

(71) Applicant: SkyLab AG, 1066 Epalinges (CH)
(72) Inventor: Belous, Elena, 121309 MOSCOW (RU); Ivanova, Angelina, LONDON, NW61NE (GB)
(74) Representative: Glawe, Delfs, Moll

(57) **Abstract**

The invention relates to a hair care composition comprising chitosan hydrochloride. Said composition protects hair from heat, increases hair thickness and/or hair density, reduces or prevents the hair from buildup of static electricity and/or electrostatic discharge, and promotes hair cuticle repair.

## Description

### THE ART TO WHICH THE INVENTION PERTAINS

The present invention relates to novel applications of a polysaccharide, chitosan hydrochloride, as an agent that contributes to heat resistance of hair, increases hair density, creates an anti-static effect and promotes hair cuticle repair. It can be used in the field of hair care products. Chitosan hydrochloride is stable and fully suitable for application in the formulation of cosmetic and pharmaceutical products.

### BACKGROUND OF THE INVENTION

Human hair represents highly structured fiber consisting of cuticle, cortex and sometimes medulla. The characteristics of these structures significantly influence the mechanical and optical properties of hair. For example, the rigidity of the central α-spiral keratin core and the abundance of disulfide bonds contribute to the remarkable resistance of hair to external influences.

Despite this rigidity, environmental conditions, pollution, chemical treatments and daily habits can result in various negative morphological and chemical changes of hair. Nowadays, usage of straightening irons and hair curlers has become a common daily practice; however, these hairstyling appliances expose hair strands to high temperatures (typically 150 through 250°C) that can break disulfide bonds. Both straightening irons and hair curlers remove moisture from the hair promoting the formation of additional protein bonds that help to change the shape of hair. The popularity of these hairstyling appliances has contributed to the emergence of a significant market for hair care products related to thermal styling, including thermal protective sprays, straightening balms, curling creams and thermal protective shampoos/conditioners. Thermal protective aids are designed to create a film on the surface of the hair, smoothing imperfections and protecting against excessive moisture loss caused by high temperatures or prolonged exposure [1].

Static electricity in hair occurs when an electrical charge builds up inside the strands. This phenomenon occurs because of friction between two dissimilar objects, which results in transfer of electrons from one object to the other. The object losing electrons acquires a positive charge and the object gaining electrons acquires a negative charge.

This process can be illustrated by the example of friction of hair against various articles of clothing. When the material rubs against the hair strands, exchange of electrons between the two surfaces occurs. As a result of this exchange, a characteristic electrical charge builds up on the hair. In conditions of higher humidity this charge is usually dissipated. And vice versa, in dry conditions, such as on a cold winter morning, the lack of moisture allows the accumulated charge to push away the hair strands from each other similar to magnetic forces.

It should be noted that electrical charges do not pass through water, which explains why the lack of humidity often observed during the winter months increases static electricity in hair. In addition, dry and damaged hair is more susceptible to static electricity due to an excess of free electrons that cannot remain stationary. This explains the pronounced static effect on such hair [2].

Different polymers are used as an effective strategy to protect hair from fragility and heating. Preliminary treatment of hair with these ingredients provides thermal protection against the breakdown of keratin in hair as well as damage to hair surface. Morphological improvement in cuticle integrity and smoothness with polymer pretreatment is of paramount importance in preventing hair fragility [3]. In a study [4], the authors examined the tryptophan content in the hair structure by means fluorescence microscopy. They determined that tryptophan is gradually destroyed during usage of curling irons at 132°C and 152°C during 4-12 minutes, and the use of polymers in preliminary treatment of the hair helps to reduce the degree of destruction by 10-20%. Patent US20020197227A1 describes the use of polymers, in particular silicones, to reduce the fuzz effect due to hair electrification [5].

Silicones are polymers and important ingredients in hair care products due to their unique properties such as improving combing, preventing damage and glossing [6]. They are intensively used to provide thermal protection to the hair [7]. Silicones are widely used in the cosmetic products industry due to their proven safety and efficacy. They are inert and do not cause allergic reactions or skin irritations, making them safe at application [8]. Silicones do not penetrate deep into the skin or hair, but remain on the surface, forming a protective permeable barrier [9].

The siloxane bond in silicones is highly strong but also very flexible, making them ideal for different hair shapes. Adding of different organic groups to the siloxane main chain gives silicones a variety of properties. They can become hydrophobic by adding of methyl groups as well as longer chain hydrocarbons, while the addition of polar groups can lead to formation of water-soluble silicone. Silicones are non-toxic and carcinogenic and do not affect the immune system of the human body. Silicones possess hypoallergenic and waterproof properties [8]. The body is unable to trap them; it is impenetrable to bacteria and does not affect the air tightness of skin and hair. Silicones are stable. They are resistant to a wide range of temperatures: from -100 to 250°C, which makes them attractive for usage as part of cosmetic products to provide thermal protection of hair; they are resistant to decomposition under the influence of ultraviolet radiation, acids, alkalis and ozone [7].

Dimethicone is one of the most popular silicones used to create long-lasting smoothness and hair glossing. It provides protection against heat damage to hair, makes it easier to comb and creates an anti-static effect [10]. The recommended concentration for use in shampoos and conditioners is 1-2% and 3-5% for serums and leave-in hair products. This silicone applies and distributes quite easily, but at high concentrations can make the hair heavier and give the effect of dirty hair [11].

Cyclopentasiloxane is a light and volatile silicone that quickly evaporates from the surface of the hair without leaving a sticky residue. It provides hair smoothness without making them heavier, giving it softness, silkiness and an anti-static effect. It is used in serums and sprays in a range of concentrations from 2 to 6%, in leave-in hair conditioners is 10%. It has only a short-term effect due to its rapid evaporation from the hair surface [12].

Aminopropyl Dimethicone is a silicone with amino groups, which has long-lasting conditioning properties and improves adhesion to the hair. It provides a long-lasting smoothness and glossing effect [13]. It is used in conditioners and masks in concentrations from 0.5 to 2%, and in leave-in hair products up to 5%. This silicone is able to improve the texture and strength of hair, suitable for damaged and colored hair, but at high concentrations can make the hair heavier and give the effect of dirty hair.

Phenyl Trimethicone has high water repellent properties that protects hair from humidity and reduces frizz, as well as gives hair a strong gloss [14]. It creates a protective barrier, preventing moisture loss. The recommended concentration for shampoos and conditioners is 1-3%, and up to 5% for leave-in hair products. It can make the hair heavier if used excessively.

Polyquaternium-37 is a novel silicone polymer that improves hair texture, combability and smoothness [15]. It provides antistatic effect and protects against mechanical damage.

Cetyl Dimethicone is a fat-soluble silicone that deeply moisturizes, nourishes and softens hair, making it more flexible and supple. It is good for dry and damaged hair, but can make the thin hair heavier [8]. It is used in conditioners and masks in the concentration of 0.5-3% and in leave-in hair products up to 5%.

Trimethicone is a light silicone that distributes easily through the hair, providing smoothness and protection from damage without making them heavier.

Dimethiconol is a silicone polymer that forms a strong protective film on the hair to prevent breakage and split ends. It provides long-lasting protection, strengthens and leaves hair glossy and smooth [16]. It is used in shampoos and conditioners at a concentration of 1-3%, and in leave-in hair products up to 5%. However, at high concentrations this silicone can make the hair heavier and give the effect of dirty hair.

Stearyl Dimethicone is a silicone that ensures excellent active moisturizing and nourishment and improves hair texture and settability of hair. It possesses softening and conditioning properties [17]. This silicone is used in conditioners and masks at the concentrations from 0.5% to 2% and in leave-in hair products up to 3%. Suitable for dry and damaged hair, but may be too heavy for thin hair [8].

Caprylyl Methicone is a volatile silicone that spreads easily; it does not make hair heavier and evaporates quickly, leaving hair soft and smooth [18]. It ensures smooth, glossy and silky hair. However, this effect is nondurable due to the component's volatility [19]. It is added in serums and sprays at a concentration of 1-5% and in leave-in hair products up to 10%.

Trifluoropropyl Dimethicone is a fluorinated silicone that ensures exceptional waterproof properties and protects hair from moisture and humidity [20]. It ensures long-term smoothness and gloss, reduces hair curling. It is added in shampoos and conditioners at a concentration of 0.5-2% and in leave-in hair products up to 3%. The disadvantage is its high cost.

Disiloxane is a volatile silicone that is used mainly as a solvent for other silicones, providing an even distribution of other ingredients. It evaporates quickly and does not leave any residues. It is a light silicone, which does not make hair heavier. It is introduced as a solvent into the product in the concentration of 1-10%, as a main component in the concentration up to 5%. It is mainly used as a solvent because of its short-term effect.

Amodimethicone represents a modified silicone that selectively accumulates on damaged areas of the hair, ensuring prolonged conditioning, moisturizing and nourishing, frizz reduction and hair thermal protection [21]. It is used in conditioners and masks at a concentration of 0.5-2% and in leave-in hair products up to 5%.

Silicones coat the hair with a thin hydrophobic (waterproof) layer. This coating reduces the porosity of the hair and they less likely will react with humidity. It also creates a smooth outer shell that reduces friction due to which each strand fits better to its neighboring strand. Smoother hair results in fewer amount of intermingled hair, as well as to and more gloss and shine. The same waterproof layer retains moisture in the hair and slows down its loss. Due to this, the effect of using the silicones containing products becomes visually striking rather quickly.

However, the waterproof protection created by silicones prevents moisture from penetrating into the hair body; so with time, tresses become dry when water evaporates and is not replenished. This waterproof is a disadvantage when trying to moisturize hair with masks and conditioners. With repeated use, silicones build up on the hair, making them heavier and result in not only a frizzy, lifeless and dull appearance, but also damage such as splitting and breakage. The silicone layer can attract dirt and other ingredients, and with time, more and more dirt builds up on the hair. It means that the active ingredients contained in conditioners and hair care products come into the hair structure with effort. Sometimes hair becomes brittle, frizzy and subject to hair shedding. Transfer to natural hair care can take up to eight washing procedures in order to remove the film of silicones that coats the hair [22].

Additional disadvantages of silicones - they are not biodegradable and introduced into products in highly enough concentrations. These polymers are synthetically derived from silicon dioxide.

The example of silicones demonstrates the potential of polymers for use in hair care products. The present invention is defined in the attached claims. The invention of the present application is focused on chitosan hydrochloride as an alternative polymer of natural origin and the claimed subject-matter finds support in terms of hard experimental data in the following examples.

### Investigations

### Example 1. In vitro determination of the efficacy of the complex for thermal hair protection

### 1.1 Materials and methods

### Hair Sample Preparation

Natural hair tresses were selected for this study, characterized by a cool white blonde coloration to ensure uniformity across all samples. Each tress was pre-treated to align with the baseline colour and texture properties, thus ensuring consistency in the assessment of treatment effects. Investigated ingredient for thermal hair protection is presented in the Table 1.

**Table 1. Investigated ingredients**

| No. | Compound |
|---|---|
| 1 | Chitosan Hydrochloride |

### 1.2 Investigation

Cuticular Surface Analysis: The cuticular morphology of the first hair tress was subjected to Scanning Electron Microscopy (SEM) to establish a baseline structural profile.

Subsequent Treatment Protocols: Two tresses were allocated for further treatment with varying concentrations of active ingredients:
An isotonic solution was prepared, containing 0.2% chitosan hydrochloride in distilled water. This preparation was adjusted to a pH range of 5-6, corresponding to hair care product standards. Hair tress number 2 was immersed in this bespoke solution for a duration of one hour.

An isotonic solution was prepared, containing 0.5% chitosan hydrochloride in distilled water. This preparation was adjusted to a pH range of 5-6, corresponding to hair care product standards. Hair tress number 3 was immersed in this bespoke solution for a duration of one hour.

The pH of all solutions was adjusted to 5.5, which is physiologically appropriate for the hair cuticle.

Thermal heating: three strands of hair: one original and two treated strands were subjected to heating using a heated flat iron used for straightening hair. The temperature of the flat iron reached 200 °C. For each strand, the flat iron was run 20 times, simulating hair straightening at home.

After a thorough rinsing of tresses numbered 2 to 4, all following samples from Table 2 was employed for a meticulous examination of the cuticular alterations by Scanning Electron Microscopy (SEM). For SEM analysis, samples were extracted from the median section of each hair tress to ensure representativeness. The microstructural examination of the acquired materials was conducted using a Tescan Amber GMH scanning electron microscope at an accelerating voltage of 1 kV, with magnifications ranging from 250x to 1000x to capture detailed topographical information of the hair cuticle.

**Table 2. Investigated systems**

| 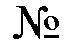 | TREATMENT GROUP | SEM IMAGES OBTAINED (QUANTITY) |
|---|---|---|
| 1 | Positive control - untreated hair | 21 |
| 2 | Negative control - thermal processing | 111 |
| 3 | 0.2% Chitosan Hydrochloride - thermal processing | 93 |
| 4 | 0.5% Chitosan Hydrochloride - thermal processing | 99 |

### 1.3 Results and discussion

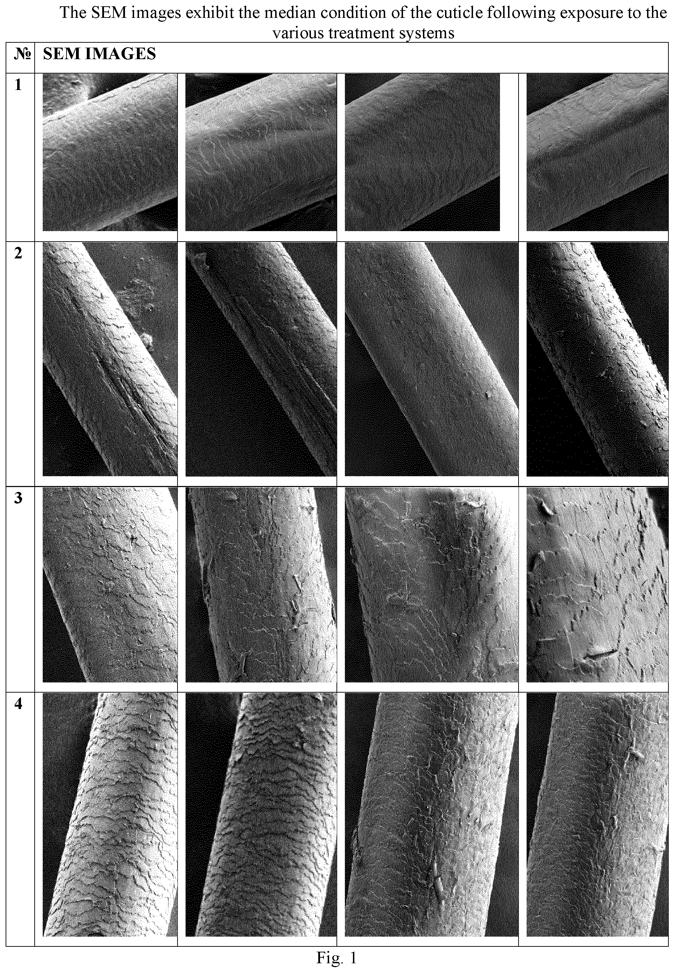

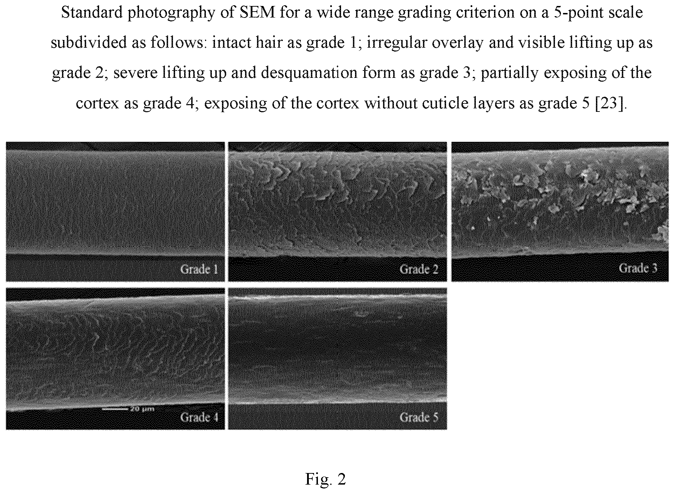

Following the Scanning Electron Microscopy (SEM) examination, a series of 18 to 25 images was captured for each sample, documenting the condition of the cuticle across a selection of hairs within the analyzed test tress. Condition of hair cuticles were analyzed using method illustrated in Figure 2. To facilitate the evaluation, the outcome data for each treatment cohort was quantified using the 'Hair Cuticle Protection Index,' where a rating of 1 denotes the optimal state of cuticle integrity and a rating of 5 represents the most compromised condition.

**Table 3. Hair cuticle protection index for treatment groups**

| 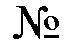 | SYSTEM | HAIR CUTICLE PROTECTION INDEX | STDEV |
|---|---|---|---|
| 1 | Positive control - untreated hair | 1.42 (p < 0.05) | 1.12 |
| 2 | Negative control - thermal processing | 2.84 (p < 0.05) | 0.45 |
| 3 | 0.2% Chitosan Hydrochloride - thermal processing | 1.90 (p < 0.05) | 0.43 |
| 4 | 0.5% Chitosan Hydrochloride - thermal processing | 1.83 (p < 0.05) | 0.38 |

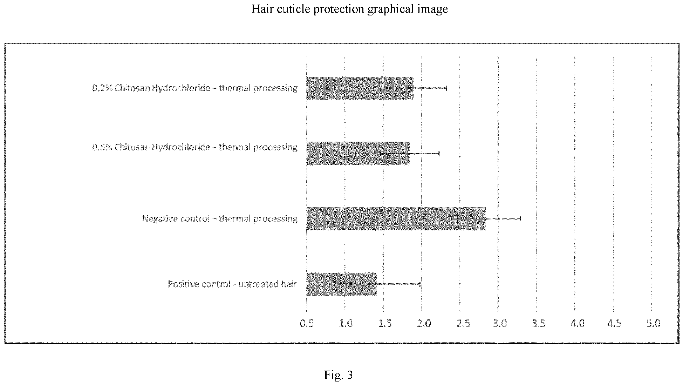

The results detailed in Table 3 and illustrated in Figure 3 demonstrate the outcomes of the study. The undamaged test strand No. 1, serving as the positive control, exhibited the healthiest cuticle condition with a mean Hair Cuticle Protection Index value of 1.4211. The degradation of test strand No. 2 by thermal processing significantly compromised the cuticle integrity, reflected in a quality index value of 2.84. Chitosan Hydrochloride, introduced as a restorative agent at two different concentrations, revealed varying efficacies. At the highest concentration of 0.5% chitosan hydrochloride, the cuticle of hair strand 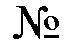 4 experienced 69.91% protection from thermal heating. At the lowest concentration of 0.2% chitosan hydrochloride, the effect of thermal heating on the hair of hair strand 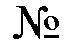 3 was reduced by 66.30%. The result indicates the effectiveness of chitosan hydrochloride as a means of protecting hair from temperature change. The advantage of chitosan hydrochloride is not only in the effective protection against heat damage to the hair, but also in its natural origin rather than synthetic like silicones.

### Example 2. In vitro determination of the efficacy of the chitosan hydrochloride for hair density increase

### 2.1 Materials and methods

### Hair Sample Preparation

Natural hair tresses were selected for this study, characterized by a cool white blonde coloration to ensure uniformity across all samples. Each tress was pre-treated to align with the baseline color and texture properties, thus ensuring consistency in the assessment of treatment effects. Investigated ingredient for hair density increase is presented in the Table 4.

**Table 4. Investigated ingredient**

| No. | Compound |
|---|---|
| 1 | Chitosan Hydrochloride |

### 2.2 Investigation

Twelve hairs were selected from the same hair strand. To measure hair thickness, an ASW (Aramo Smart Wizard) - Aram Human Vision System was used for skin (dermatoscopy) and hair (trichoscopy) diagnosis under magnification. Five measurements were taken from each hair before further manipulation. The average hair thickness was used for further calculations of hair thickness «before» manipulation.

An aqueous solution with a 0.25% concentration of a commercially sourced protease was prepared, involving the dissolution of 0.75 grams of the enzyme in 300 millilitres of distilled water, followed by homogenization to ensure uniform distribution. 12 hairs from the previous step were submerged in the proteolytic bath. The solution was agitated at 15-minute intervals using a Pasteur pipette over a period of one hour to facilitate enzymatic penetration and action.

All hairs were then dried. The thickness of each hair was measured as before five times. The average thickness of the fractured hairs was measured as before five times.

All hairs were then dried. The thickness of each hair was measured as before five times. The average thickness of protease-depleted hair was used for further comparison.

Subsequent Treatment Protocols: Hairs were allocated for further treatment with varying concentrations of active ingredients:
An isotonic solution was prepared, containing 0.2% chitosan hydrochloride in distilled water. This preparation was adjusted to a pH range of 5-6, corresponding to hair care product standards. Six hairs were immersed in this bespoke solution for a duration of one hour.

An isotonic solution was prepared, containing 0.5% chitosan hydrochloride in distilled water. This preparation was adjusted to a pH range of 5-6, corresponding to hair care product standards. Six hairs were immersed in this bespoke solution for a duration of one hour.

The pH of all solutions was adjusted to 5.5, which is physiologically appropriate for the hair cuticle.

All hairs were dried at room temperature. The thickness of each hair was measured five times. The average thickness was used for further comparison.

### 2.3 Results and discussion

**Table 5. Hair density for treatment groups**

| 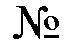 | SYSTEM | HAIR DENSITY, MM | STDEV |
|---|---|---|---|
| 1 | Positive control - untreated hair | 0.3093 (p < 0.05) | 0.0371 |
| 2 | Negative control - treated with 0.25% protease | 0.2264 (p < 0.05) | 0.0135 |
| 3 | 0.2% Chitosan Hydrochloride | 0.2837 (p < 0.05) | 0.0265 |
| 4 | 0.5% Chitosan Hydrochloride | 0.2993 (p < 0.05) | 0.0304 |

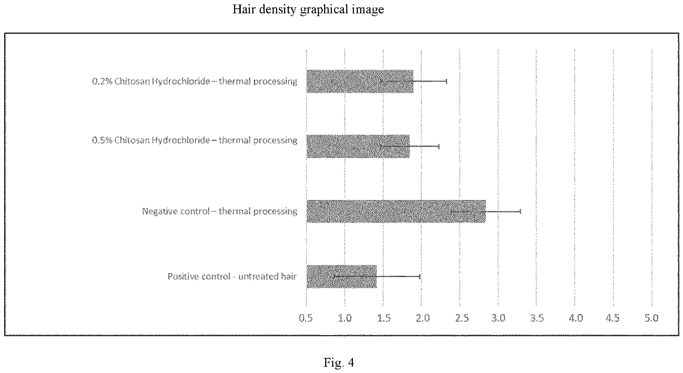

The results shown in Table 5 and illustrated in Figure 4 demonstrate the outcome of the study. Intact hair, which served as a positive control, demonstrated an average hair thickness of 0.31 mm. Hair degradation as a result of the 0.25% protease treatment significantly disrupted the integrity of the cuticle, which was reflected in reduced hair thickness. The average thickness of the degraded hair was 0.23 mm. Chitosan hydrochloride introduced as a restorative agent at two different concentrations showed different efficacy. At the highest concentration of 0.5% chitosan hydrochloride, hair thickness recovered 69.12% after treatment. At the lowest concentration of 0.2% chitosan hydrochloride, hair thickness recovered by 87.94%. The obtained result indicates the effectiveness of chitosan hydrochloride as a means of increasing hair density and can be used as a natural analogue of silicones.

Due to the thickening of the hair as a result of the treatment with chitosan hydrochloride, we assume that the thermal protection of the hair and the anti-static effect are provided by the formation of a film of this polymer on the hair surface.

### Example 3. Investigation of Anti-Electrostatic and Mechanical Properties of the chitosan hydrochloride

### 3.1 Materials and methods

### Hair Sample Preparation

Natural hair tresses were selected for this study, characterized by a cool white blonde coloration to ensure uniformity across all samples. Each tress was pre-treated to align with the baseline colour and texture properties, thus ensuring consistency in the assessment of treatment effects. Investigated ingredient for thermal hair protection is presented in the Table 6.

**Table 6. Investigated ingredients**

| No. | Compound |
|---|---|
| 1 | Chitosan Hydrochloride |

As a control, a strand of hair that had not been pre-treated with anything was used. This strand was labelled 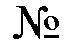 1.

Subsequent Treatment Protocols: Two tresses were allocated for further treatment with varying concentrations of active ingredients:
An isotonic solution was prepared, containing 0.2% chitosan hydrochloride in distilled water. This preparation was adjusted to a pH range of 5-6, corresponding to hair care product standards. Hair tress number 2 was immersed in this bespoke solution for a duration of one hour.

An isotonic solution was prepared, containing 0.5% chitosan hydrochloride in distilled water. This preparation was adjusted to a pH range of 5-6, corresponding to hair care product standards. Hair tress number 3 was immersed in this bespoke solution for a duration of one hour.

The pH of all solutions was adjusted to 5.5, which is physiologically appropriate for the hair cuticle.

The anti-electrostatic performance of the hair samples was analyzed using an air filled rubber balloon that was stroked thrice with a cotton fabric. Thereafter, the ballloon was positioned near the hair samples, and electrostatic phenomena were captured using a camera.

### 3.2. Results

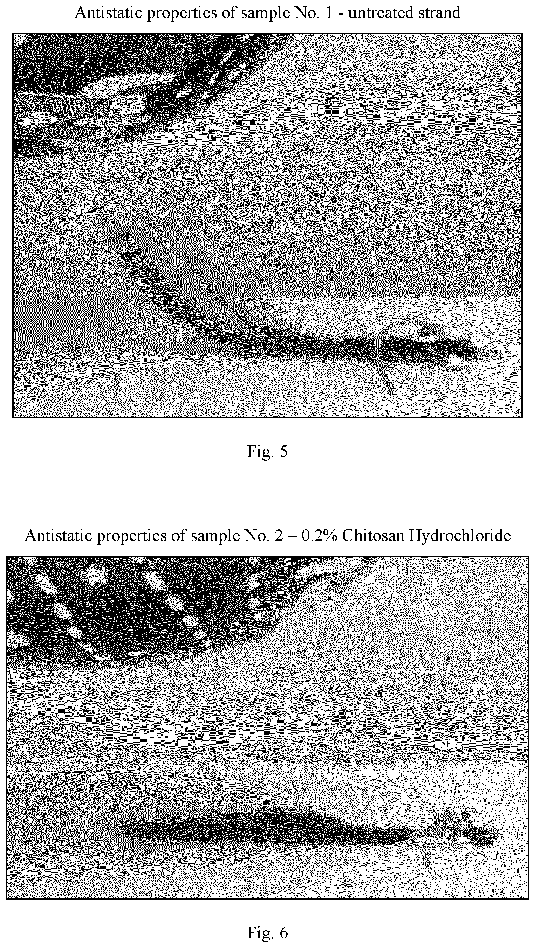

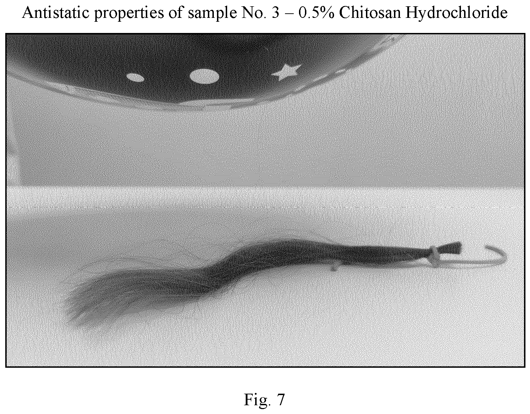

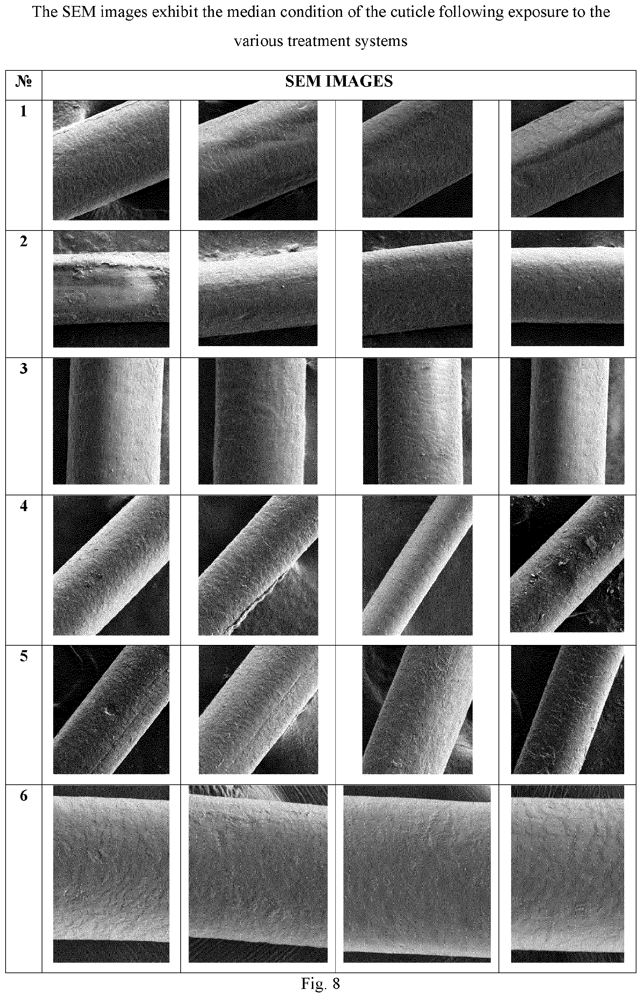

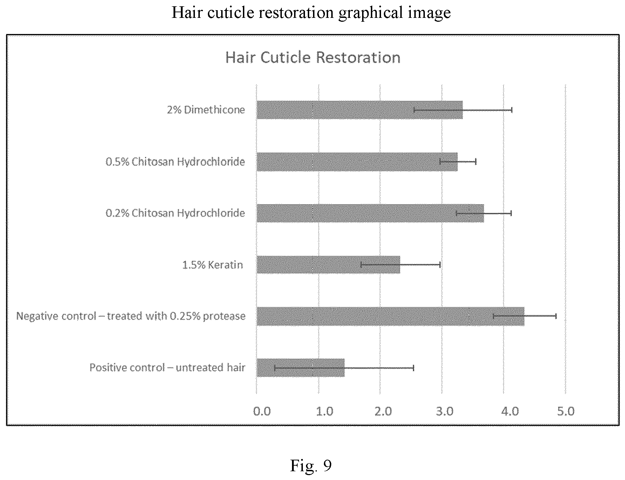

The occurrence of electrostatic charges on the hair triggers entanglement and repulsion between hair molecules, damaging the hair and causing inconvenience. Therefore, the antistatic property of chitosan hydrochloride plays an important role in addressing this issue by dissipating electrostatic charges on the hair. To observe the antistatic effects, we compared chitosan hydrochloride-treated hair with untreated hair that was placed near a rubber balloon. As shown in Figures 6 and 7, the chitosan hydrochloride-treated hair contain displayed a notable anti static effect. This was evident, as the hair did not cling to the rubber balloon because of static induction. In contrast, the untreated hair adhered to the rubber balloon, indicating a lack of antistatic properties. This result suggests that the dissipation of electrostatic charges on the chitosan hydrochloride-treated hair was attributable to stacking, which can increase conductivity, thereby facilitating effective charge dissipation on the hair. The hair may have also been affected by the increase in hydrophilicity after its coating with chitosan hydrochloride, which reduced surface resistivity on the hair [24,25].

### Example 4. In vitro determination of the efficacy of the complex for hair cuticle restoration

### 4.1 Materials and methods

### Hair Sample Preparation

Natural hair tresses were selected for this study, characterized by a cool white blonde coloration to ensure uniformity across all samples. Each tress was pre-treated to align with the baseline color and texture properties, thus ensuring consistency in the assessment of treatment effects. Commercial protease was bought from Creative Enzymes, USA as an ingredient for targeted destruction of hair cuticle. Investigated ingredients for hair restoration presented in the Table 7.

**Table 7. Investigated ingredients**

| No. | Compound |
|---|---|
| 1 | Chitosan Hydrochloride |
| 2 | Keratin |
| 3 | Dimethicone |

### 4.2 Investigation

Cuticular Surface Analysis: The cuticular morphology of the first hair tress was subjected to Scanning Electron Microscopy (SEM) to establish a baseline structural profile.

Proteolytic Solution Formulation: An aqueous solution with a 0.25% concentration of a commercially sourced protease was prepared, involving the dissolution of 0.75 grams of the enzyme in 300 milliliters of distilled water, followed by homogenization to ensure uniform distribution.

Proteolytic Treatment Incubation: The remaining four tresses (excluding the first) were submerged in the proteolytic bath. The solution was agitated at 15-minute intervals using a Pasteur pipette over a period of one hour to facilitate enzymatic penetration and action.

Post-Treatment Rinse and SEM Assessment: Post enzymatic exposure, tresses numbered 2 through 6 were lavaged with copious amounts of water. Subsequent to this, the second hair tress underwent SEM to evaluate the enzymatic impact on cuticular integrity.

Subsequent Treatment Protocols: Four tresses from the previous step were allocated for further treatment with varying concentrations of active ingredients:
As a positive control a solution with 1.5% keratin was prepared. Keratin was used as a well-known ingredient for hair restoration which is biomimetic to hair cuticle. Hair tress number 3 was immersed in this bespoke solution for a duration of one hour.

An isotonic solution was prepared, containing 0.2% chitosan hydrochloride in distilled water. This preparation was adjusted to a pH range of 5-6, corresponding to hair care product standards. Hair tress number 4 was immersed in this bespoke solution for a duration of one hour.

An isotonic solution was prepared, containing 0.5% chitosan hydrochloride in distilled water. This preparation was adjusted to a pH range of 5-6, corresponding to hair care product standards. Hair tress number 5 was immersed in this bespoke solution for a duration of one hour.

An isotonic solution was prepared, containing 2% dimethicone in distilled water to compare hair texture restoration with chitosan hydrochloride and with commonly used silicone. This preparation was adjusted to a pH range of 5-6, corresponding to hair care product standards. Hair tress number 5 was immersed in this bespoke solution for a duration of one hour.

The pH of all solutions was adjusted to 5.5, which is physiologically appropriate for the hair cuticle.

After a thorough rinsing of tresses numbered 3 to 6, all following samples from Table 7 was employed for a meticulous examination of the cuticular alterations post-chemical treatment by Scanning Electron Microscopy (SEM). For SEM analysis, samples were extracted from the median section of each hair tress to ensure representativeness. The microstructural examination of the acquired materials was conducted using a Tescan Amber GMH scanning electron microscope at an accelerating voltage of 1 kV, with magnifications ranging from 250x to 1000x to capture detailed topographical information of the hair cuticle.

**Table 8. Investigated systems**

| 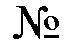 | TREATMENT GROUP | SEM IMAGES OBTAINED (QUANTITY) |
|---|---|---|
| 1 | Positive control - untreated hair | 21 |
| 2 | Negative control - treated with protease | 21 |
| 3 | 1.5% Keratin | 18 |
| 4 | 0.2% Chitosan Hydrochloride | 25 |
| 5 | 0.5% Chitosan Hydrochloride | 20 |
| 6 | 2% Dimethicone | 39 |

### 4.3 Results and discussion

Following the Scanning Electron Microscopy (SEM) examination, a series of 18 to 39 images was captured for each sample, documenting the condition of the cuticle across a selection of hairs within the analyzed test tress. Condition of hair cuticles were analyzed using method illustrated in Figure 2. To facilitate the evaluation, the outcome data for each treatment cohort was quantified using the 'Hair Cuticle Restoration Index,' where a rating of 1 denotes the optimal state of cuticle integrity and a rating of 5 represents the most compromised condition.

**Table 9. Hair cuticle restoration index for treatment groups**

| 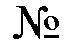 | SYSTEM | HAIR CUTICLE RESTORATION INDEX | STDEV |
|---|---|---|---|
| 1 | Positive control - untreated hair | 1.42 (p < 0.05) | 1.12 |
| 2 | Negative control - treated with 0.25% protease | 4.32 (p < 0.05) | 0.51 |
| 3 | 1.5% Keratin | 2.33 (p < 0.05) | 0.64 |
| 4 | 0.2% Chitosan Hydrochloride | 3.68 (p < 0.05) | 0.44 |
| 5 | 0.5% Chitosan Hydrochloride | 3.26 (p < 0.05) | 0.29 |
| 6 | 2% Dimethicone | 3.34 (p < 0.05) | 0.79 |

The results detailed in Table 8 and illustrated in Figure 6 demonstrate the outcomes of the study. The undamaged test strand No. 1, serving as the negative control, exhibited the healthiest cuticle condition with a mean Hair Cuticle Restoration Index value of 1.4211. The degradation of test strand No. 2 by protease significantly compromised the cuticle integrity, reflected in a quality index value of 4.32. Keratin, employed as a market reference biomimetic to hair for restorative purposes, yielded a cuticle recovery result of 2.33, equating to a 64% restoration. Chitosan Hydrochloride, introduced as a restorative agent at two different concentrations, revealed varying efficacies. At the highest concentrations used-0.5% Chitosan Hydrochloride-the cuticle of hair strand No. 5 experienced a 43.67% restoration. The lowest concentrations of 0.2% for chitosan hydrochloride led to an 30.44% cuticle restoration of hair strand No. 4. The result demonstrates less effectiveness of chitosan hydrochloride than keratin in hair restoration. However, the presence of the cuticle repair property complements the other stronger properties. At the same time, animal keratin is used in hair products, while chitosan hydrochloride is vegan because it is derived from mushrooms.

The results obtained for dimethicone were comparable to the recovery results obtained for chitosan hydrochloride. The advantage of the latter is the natural origin and the activity of the component at a much lower concentration.

An important aspect of the study results is the cost-effectiveness related to the concentration of the preparation: a 0.2-0.5% composition of active ingredients is less effective than 1.5% keratin, but can potentially affect the price of the final product. In addition, the system under investigation is vegan, unlike keratin, which does not have vegan or vegetarian certification.

### LITERATURE

1. Tinoco A, Goncalves J, Silva C, Cavaco-Paulo A, Ribeiro A. Crystallin Fusion Proteins Improve the Thermal Properties of Hair. Front Bioeng Biotechnol. 2019 Oct 25;7:298. doi: 10.3389/fbioe.2019.00298. PMID: 31709253; PMCID: PMC6823552.
2. Draelos Z. D. Hair care: an illustrated dermatologic handbook. - CRC Press, 2004.
3. Zhou Y, Rigoletto R, Koelmel D, Zhang G, Gillece TW, Foltis L, Moore DJ, Qu X, Sun C. The effect of various cosmetic pretreatments on protecting hair from thermal damage by hot flat ironing. J Cosmet Sci. 2011 Mar-Apr;62(2):265-82. PMID: 21635854.
4. McMullen R., Jachowicz J. Thermal degradation of hair. II. Effect of selected polymers and surfactants //Journal of the Society of Cosmetic Chemists. - 1998. - T. 49. - 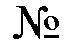 . 4. - C. 245-256.
5. Patent US20020197227A1. Anti-frizz hair care compositions URL: https://patents.google.com/patent/US20020197227A1/en
6. Gavazzoni Dias MF. Hair cosmetics: an overview. Int J Trichology. 2015 Jan-Mar;7(1):2-15. doi: 10.4103/0974-7753.153450. PMID: 25878443; PMCID: PMC43 87693 .
7. Patent WO2017040876A1. Silicones for heat protection URL: https://patents.google.com/patent/WO2017040876A1/en
8. Bains P, Kaur S. Silicone in Dermatology: An Update. J Cutan Aesthet Surg. 2023 Jan-Mar;16(1):14-20. doi: 10.4103/JCAS.JCAS_204_22. Epub 2023 Apr 28. PMID: 37383974; PMCID: PMC10298615.
9. Ivanova, E.V., Minyaylo, E.O., Temnikov, M.N. et al. Silicones in Cosmetics. Polym. Sci. Ser. B 65, 578-594 (2023). https://doi.org/10.1134/S1560090423600201
10. Bains P, Kaur S. Silicone in Dermatology: An Update. J Cutan Aesthet Surg. 2023 Jan-Mar;16(1):14-20. doi: 10.4103/JCAS.JCAS_204_22. Epub 2023 Apr 28. PMID: 37383974; PMCID: PMC10298615.
11. Madnani N, Khan K. Hair cosmetics. Indian J Dermatol Venereol Leprol. 2013 Sep-Oct;79(5):654-67. doi: 10.4103/0378-6323.116734. PMID: 23974582.
12. Dudzina T, Garcia Hidalgo E, von Goetz N, Bogdal C, Hungerbuehler K. Evaporation of decamethylcyclopentasiloxane (D5) from selected cosmetic products: Implications for consumer exposure modeling. Environ Int. 2015 Nov;84:55-63. doi: 10.1016/j.envint.2015.07.013. Epub 2015 Jul 26. PMID: 26222996.
13. Patent EP3687485A1. Hydrous hair care compositions and methods URL: https://patents.google.com/patent/EP3687485A1/en
14. Patent EP1543814A1. Silicone-free hair care compositions providing long-lasting shine URL: https://patents.google.com/patent/EP1543814A1/en
15. Patent EP1779838A1. Hair conditioning composition comprising Polyquaternium 37, fatty alcohol and non-ionic / cationic surfactant URL: https://patents.google.com/patent/EP1779838A1/en
16. Patent KR20190002835A. Hair conditioning composition URL: https://patents.google.com/patent/KR20190002835A/en
17. O'Lenick Jr A. J., O'Lenick K. A. Silicone polymers in skin care //MRS bulletin. - 2007. - T. 32. - 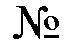 . 10. - C. 801-806.
18. Linda Fleming et al. LIMONENYL TRISILOXANE: Naturalized Replacement for Caprylyl Methicone in Personal Care Applications. NYSCC tech conf 2015 Dec. doi: 10. 13 140/RG. 2.2.3 3 166.5 6646
19. Molinier B, Arata C, Katz EF, Lunderberg DM, Liu Y, Misztal PK, Nazaroff WW, Goldstein AH. Volatile Methyl Siloxanes and Other Organosilicon Compounds in Residential Air. Environ Sci Technol. 2022 Nov 15;56(22):15427-15436. doi: 10.1021/acs.est.2c05438. Epub 2022 Nov 3. PMID: 36327170; PMCID: PMC9670844.
20. Patent WO2007079793A1. Care hair-treatment composition with hyperbranched polymers URL: https://patents.google.com/patent/WO2007079793A1/en
21. D'Souza P, Rathi SK. Shampoo and Conditioners: What a Dermatologist Should Know? Indian J Dermatol. 2015 May-Jun;60(3):248-54. doi: 10.4103/0019-5154.156355. PMID: 26120149; PMCID: PMC4458934.
22. WHY WE DON'T EVER USE SILICONES IN OUR PRODUCTS / LoveNoughty. URL: https://lovenoughty.co.uk/blogs/news/why-we-dont-ever-use-silicones-in-our-products (18.10.2024).
23. Lee S. Y. et al. Twelve-point scale grading system of scanning electron microscopic examination to investigate subtle changes in damaged hair surface //Skin Research and Technology. - 2016. - T. 22. - 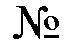 . 4. - C. 406-411
24. Kim TM, Won HJ, Yang JH, Jo H, Kim AH, Nam D, Kim SG, Jin EJ, Bae HJ, Park SY. Multicolor Hair Dyeing with Biocompatible Dark Polyphenol Complex-Integrated Shampoo with Reactive Oxygen Species Scavenging Activity. Biomimetics (Basel). 2023 Oct 1;8(6):469. doi: 10.3390/biomimetics8060469. PMID: 37887600; PMCID: PMC10604431.
25. Won HJ, Kim TM, An IS, Bae HJ, Park SY. Protection and Restoration of Damaged Hair via a Polyphenol Complex by Promoting Mechanical Strength, Antistatic, and Ultraviolet Protection Properties. Biomimetics (Basel). 2023 Jul 9;8(3):296. doi: 10.3390/biomimetics8030296. PMID: 37504184; PMCID: PMC10807499.

## Claims

1. A heat protecting and/or antistatic hair care composition comprising chitosan hydrochloride.

2. The composition of claim 1, wherein said composition has a pH in the range of 4.0 - 9.0, preferably in the range of 5-6, further preferably 5.5.

3. The composition of claim 1, wherein said chitosan hydrochloride is present in said composition in an amount of at least 0.10% by weight of said composition, preferably at least 0.15% by weight, further preferably at least 0.20% by weight or in an amount of a range selected from the following: 0.10-10.00% by weight, 0.10-5.00% by weight, 0.10-3.00% by weight, 0.10-1.00% by weight, 0.10-0.75 % by weight, 0.10-0.50% by weight, 0.15-10.00% by weight, 0.15-5.00% by weight, 0.15-3.00% by weight, 0.15-1.00% by weight, 0.15-0.75% by weight, 0.15-0.50% by weight, 0.20-10.00% by weight, 0.20-5.00% by weight, 0.20-3.00% by weight, 0.20-1.00% by weight, 0.20-0.75% by weight, 0.20-0.50% by weight.

4. The composition of any of the preceding claims, said composition not comprising a detersive component.

5. The composition of any of the preceding claims, said composition not comprising
i) a silicone or polysiloxane compound, and/or
ii) keratin.

6. The composition of any of the preceding claims, wherein said composition is formulated for application on a subject's hair, wherein preferably said composition is a formulation selected from the following: liquid, aerosol, suspension, solution, tincture, cream, paste, lotion, ointment, gel, balsam, foam, shampoo and conditioner.

7. The use of a hair care composition comprising chitosan hydrochloride for at least one of the following uses or any combination thereof:
i) protecting hair from heat,
ii) increasing hair thickness and/or hair density,
iii) reducing or preventing the hair from buildup of static electricity and/or electrostatic discharge, and
iv) promoting hair cuticle repair.

8. The use of claim 7, wherein said composition has a pH in the range of 4.0 - 9.0, preferably in the range of 5-6, further preferably 5.5.

9. The use of claim 7 or claim 8, wherein said chitosan hydrochloride is present in said composition in an amount of at least 0.10% by weight of said composition, preferably at least 0.15% by weight, further preferably at least 0.20% by weight or in an amount of a range selected from the following: 0.10-10.00% by weight, 0.10-5.00% by weight, 0.10-3.00% by weight, 0.10-1.00% by weight, 0.10-0.75 % by weight, 0.10-0.50% by weight, 0.15-10.00% by weight, 0.15-5.00% by weight, 0.15-3.00% by weight, 0.15-1.00% by weight, 0.15-0.75% by weight, 0.15-0.50% by weight, 0.20-10.00% by weight, 0.20-5.00% by weight, 0.20-3.00% by weight, 0.20-1.00% by weight, 0.20-0.75% by weight, 0.20-0.50% by weight.

10. The use of any of claims 7-9, said composition not comprising a detersive component.

11. The use of any of claims 7-10, said composition not comprising
i) a silicone or polysiloxane compound, and/or
ii) keratin.

12. The use of any of claims 7-11, wherein said composition is formulated for application on a subject's hair, wherein preferably said composition is a formulation selected from the following: liquid, aerosol, suspension, solution, tincture, cream, paste, lotion, ointment, gel, balsam, foam, shampoo and conditioner.

13. A method of protecting hair from heat and/or for reducing or preventing the hair from buildup of static electricity and/or electrostatic discharge, said method comprising treating the hair with a composition of any of claims 1-6.

14. The method of claim 13, wherein said hair is protected from heat temperatures up to 200 °C, preferably up to 170 °C.

15. The method of claim 13 or claim 14, wherein said composition is applied to said hair less than 30 minutes to application of the heat to said hairs.
